(19) Europäisches Patentamt
European Patent Office
Office européen des brevets

(11) Publication number: **0 150 891 A1**

(12) **EUROPEAN PATENT APPLICATION**

(21) Application number: **85300011.5**

(22) Date of filing: **02.01.85**

(51) Int. Cl.⁴: **C 07 D 335/16**, C 07 D 409/04, A 61 K 31/38

(30) Priority: **05.01.84 GB 8400201**
**05.01.84 GB 8400202**
**05.01.84 GB 8400203**

(43) Date of publication of application: **07.08.85**
**Bulletin 85/32**

(84) Designated Contracting States: **AT BE CH DE FR GB IT LI LU NL SE**

(71) Applicant: **THE WELLCOME FOUNDATION LIMITED, 183-193 Euston Road, London NW1 2BP (GB)**

(72) Inventor: **Harfenist, Morton, Route 6, Box 405, Chapel Hill North Carolina 27514 (US)**
Inventor: **Joyner, Charles Thomas, Route 8 Box 183H, Raleigh North Carolina 27612 (US)**
Inventor: **Heuser, Darryl James, 208 Blanchard Street, Raleigh North Carolina 27603 (US)**

(74) Representative: **Garrett, Michael et al, The Wellcome Foundation Limited Group Patents and Agreements Langley Court, Beckenham Kent BR3 3BS (GB)**

(54) **Tricyclic compounds, processes for their preparation, compositions containing such compounds and their use in medicine.**

(57) Compounds of formula (I)

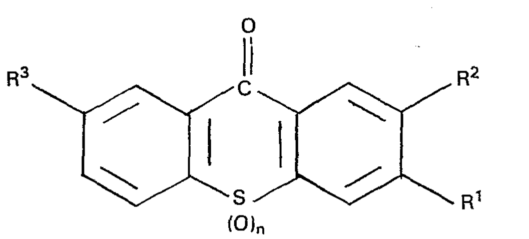

(I')

wherein n is 0, 1 or 2;
one of $R^1$ and $R^2$ is hydrogen and the other is selected from carbamoyl, N-$C_{1-4}$ alkylcarbamoyl, N,N-di-$C_{1-4}$ alkylcarbamoyl, carbamimidoyl, $N^1$-$C_{1-4}$ alkylcarbamimidoyl, $N^2$-$C_{1-4}$ alkylcarbamimidoyl, $N^1$-$C_{1-4}$ alkyl-$N^2$-$C_{1-4}$ alkylcarbamimidoyl, $N^1$,$N^1$-di-$C_{1-4}$ alkylcarbamimidoyl, $N^1$,$N^1$-di-$C_{1-4}$alkyl-$N^2$-$C_{1-4}$ alkylcarbamimidoyl, tetrazol-5-yl (optionally substituted by one or more $C_{1-4}$ alkyl groups) and imidazolin-2-yl (optionally substituted by one or more $C_{1-4}$ alkyl groups); and
$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from hydrogen, $C_{1-4}$ alkyl and hydroxy $C_{1-4}$ alkyl, provided that the total number of carbon atoms in $R^5$ and $R^6$ does not exceed 4), amino $C_{1-4}$ alkylamino and morpholino,
provided that when $R^1$ is carbamoyl and $R^2$ and $R^3$ are both hydrogen, then n is 0 or 1; and physiologically acceptable salts thereof, and their pro-drugs and metabolites are inhibitors of monoamine oxidase-A and are useful in the prophylaxis and treatment of mental disorders such as depression.

EP 0 150 891 A1

# TRICYCLIC COMPOUNDS, PROCESSES FOR THEIR PREPARATION, COMPOSITIONS CONTAINING SUCH COMPOUNDS AND THEIR USE IN MEDICINE

The present invention relates to tricyclic compounds having valuable monoamine oxidase inhibitory activity.

Monoamine oxidase (MAO) is a brain enzyme believed to be responsible for intraneuronal catalysis of oxidation of biogenic amine neurotransmitters to inactive forms. It is understood to occur as two independent enzymes normally designated MAO-A and MAO-B (White and Glassman, J. Neurochem., 29, 987-997, (1977) and Tipton et al, "Monoamine Oxidase and its Selective Inhibitors", Beckmann and Riederer, Eds., Mod. Probl. Pharmacopsychiat., 19 15-30, Karger, Basel (1983)). MAO inhibition has been found to elevate neurotransmitter concentration in the brain. MAO inhibitors are used therapeutically in the treatment of a wide variety of conditions, especially depression, particularly when characterized by anxiety, obessional neuroses, or appetite disorders. However, many known MAO inhibitors such as phenelzine have an undesirable side effect associated with ingestion of food or drink containing tyramine, for example certain cheeses. When a patient receiving a conventional MAO inhibitor ingests a tyramine containing product, then his blood pressure may be raised, sometimes to a dangerous level. Such patients are therefore instructed to avoid certain foods.

In M.Harfenist, J.Med.Chem. 23 (1980) 825-7, there is described a compound 3-acetamidothioxanthen-9-one 10,10-dioxide (hereinafter termed the known compound) having MAO inhibitory activity. However, the known compound is an arylamide derivative and it is known that many arylamines and arylamides, as well as compounds which are, metabolised thereto, have significant carcinogenicity (eg. see Charles C. Irving, Aromatic Amines and Azo Dyes, in Proc. 2nd FDA Symp. on structural Correlates of Carcinogenesis and Mutagenesis, US Naval Academy, 1977, pp 136 ff). In general, arylamides and their derivatives are prone to cause bone marrow depression, methaemaglobin formation and other toxic effects. Moreover, the known compound is structurally related to the known carcinogen 2-acetyl-aminofluorene. Such toxicity is especially undesirable in compounds likely to be administered repeatedly over prolonged periods, as is normally anticipated for the prevention or alleviation of the symptoms of chronic mental disorders. The known compound has also shown weak activity in animal models predictive of antidepressant activity in man.

Other thioxanthen-9-one 10, 10-dioxide derivatives are also known as therapeutic agents, for example as described in US patent specification nos. 4 091 108 and 3 905 989, and in UK patent specification no. 3 905 989 (hereinafter termed the "Hodson and Batchelor specifications"). However the latter class of compounds are only disclosed as anti-allergic agents and there is no suggestion that they might possess useful MAO-inhibitory activity. Included in this class is the compound 3-carbamoyl-7-methylthioxanthen-9-one 10, 10-dioxide.

We have now discovered that the compounds of formula (I) as defined hereinbelow have shown unexpectedly superior potency relative to that of the known compound when tested in a screen predictive of anti-depresent activity. Moreover, since many of the compounds of formula (I) are not arylamines or arylamides, they do not carry such a potential risk of carcinogenicity and the other toxic effects.

Thus the present invention provides compounds of formula (I)

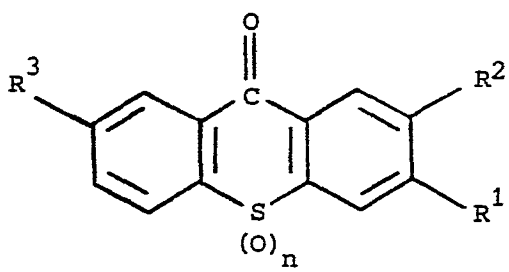

(I)

wherein n is 0, 1 or 2;

one of $R^1$ and $R^2$ is hydrogen and the other is selected from carbamoyl, N-$C_{1-4}$ alkylcarbamoyl, N,N-di-$C_{1-4}$alkylcarbamoyl, carbamimidoyl, $N^1$-$C_{1-4}$ alkylcarbamimidoyl, $N^2$-$C_{1-4}$alkylcarbamimidoyl, $N^1$-$C_{1-4}$alkyl-$N^2$-$C_{1-4}$alkyl-carbamimidoyl, $N^1$, $N^1$-di-$C_{1-4}$alkylcarbamimidoyl, $N^1$, $N^1$-di-$C_{1-4}$alkyl-$N^2$-$C_{1-4}$alkylcarbamimidoyl, tetrazol-5-yl (optionally substituted by one or more $C_{1-4}$ alkyl groups) and imidazolin-2-yl (optionally substituted by one or more $C_{1-4}$ alkyl groups); and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from hydrogen, $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

provided that when $R^1$ is carbamoyl, $R^2$ is hydrogen and $R^3$ is hydrogen or methyl, or when $R^1$ is 2-methyltetrazol-5-yl and $R^2$ and $R^3$ are both hydrogen, then n is 0 or 1; and

when $R^1$ is unsubstituted tetrazol-5-yl, $R^2$ is hydrogen and $R^3$ is selected from hydrogen, chlorine, methyl, ethyl and t-butyl, then n is 1.

Included within the meaning of the compounds of formula (I) are the physiologically acceptable salts of such compounds. Thus, unless the context requires otherwise, any reference herein to one or more compounds of formula (I) is to be taken, equally, as a reference to physiologically acceptable salts thereof.

The present invention also includes pro-drugs and metabolites of the compounds of formula (I) and their physiologically acceptable salts. By pro-drugs is meant those compounds which are metabolised in vivo to form a compound of formula (I) or a physiologically acceptable salt thereof. By metabolite is meant any compound resulting directly or indirectly from the in vivo metabolism of a compound of formula (I) or a physiologically acceptable salt thereof.

Certain thioxanthen-9-ones, and their 10-oxides and 10,10-dioxides, said to be anti-allergic agents, are described respectively in UK patent specification 1 458 185 and US patent specifications 4 091 108 and 3 905 989 (hereinafter referred to as the 'Batchelor and Hodson Specifications'). We have also discovered that as well as the compounds of formula (I), some compounds disclosed in the Batchelor and Hodson specifications are also unexpectedly advantageous MAO-A inhibitors. The aforementioned known compounds and the compounds of formula (I), together constitute a class of compounds of formula (I') and their pharmacologically acceptable salts. In formula (I')

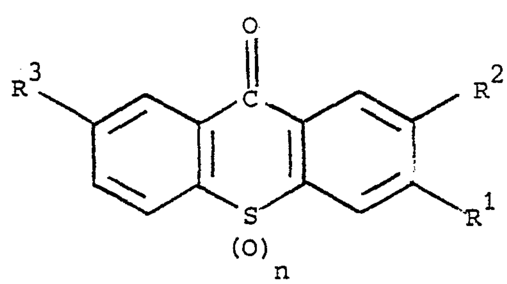

(I')

n is 0, 1 or 2;

one of $R^1$ and $R^2$ is hydrogen and the other is selected from carbamoyl, N-$C_{1-4}$ alkylcarbamoyl, N,N-di-$C_{1-4}$alkylcarbamoyl, carbamimidoyl, $N^1$-$C_{1-4}$ alkylcarbamimidoyl, $N^2$-$C_{1-4}$alkylcarbamimidoyl, $N^1$-$C_{1-4}$alkyl-$N^2$-$C_{1-4}$

alkylcarbamimidoyl, $\underline{N}^1$, $\underline{N}^1$-di-$C_{1-4}$alkylcarbamimidoyl, $\underline{N}^1$, $\underline{N}^1$-di-$C_{1-4}$alkyl-$\underline{N}^2$-$C_{1-4}$alkylcarbamimidoyl, tetrazol-5-yl (optionally substituted by one or more $C_{1-4}$ alkyl groups) and imidazolin-2-yl (optionally substituted by one or more $C_{1-4}$ alkyl groups); and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from hydrogen, $C_{1-4}$ alkyl and hydroxy $C_{1-4}$ alkyl, provided that the total number of carbon atoms in $R^5$ and $R^6$ does not exceed 4), amino-$C_{1-4}$ alkylamino and morpholino;

provided that when $R^1$ is carbamoyl and $R^2$ and $R^3$ are both hydrogen, then n is 0 or 1.

Included within the scope of the compounds of formula (I') are also the physiologically acceptable salts of such compounds. In particular, the latter salts and also the pharmocologically acceptable salts of compounds of formula (I) may be the acid addition salts of compounds wherein one of $R^1$ and $R^2$ represents a carbamimidoyl, $\underline{N}^1$-$C_{1-4}$ alkylcarbamimidoyl, $\underline{N}^2$-$C_{1-4}$alkylcarbamimidoyl, $\underline{N}^1$-$C_{1-4}$alkyl-$\underline{N}^2$-$C_{1-4}$alkylcarbamimidoyl, $\underline{N}^1$, $\underline{N}^1$-di-$C_{1-4}$alkylcarbamimidoyl, $\underline{N}^1$, $\underline{N}^1$-di-$C_{1-4}$alkyl-$\underline{N}^2$- $C_{1-4}$alkylcarbamimidoyl or an optionally alkylated imidazolin-2-yl group and/or $R^3$ represents a group of formula $-NR^5R^6$, an amino -$C_{1-4}$ alklamino group or a morpholino group, for example those derived from hydrochloric, hydrobromic, phosphoric, malic, maleic, fumaric, citric, sulphuric, lactic and tartaric acids.

In formulae (I) and (I'), when $R^3$ and/or $R^4$ is a saturated or unsaturated $C_{1-4}$ aliphatic hydrocarbon moiety, these groups may be selected independently from, for example $C_{1-4}$ alkyl, $C_{2-4}$ alkenyl and $C_{2-4}$ alkynyl groups.

A preferred sub-class of the compounds of formula (I) comprises those compounds wherein $R^1$ is hydrogen, $R^2$ represents an imidazolin-2-yl group or an $\underline{N}$-$C_{1-4}$alkylcarbamoyl group (especially $\underline{N}$-methylcarbamoyl) and $R^3$ represents a group selected from $C_{1-4}$ alkyl (especially isopropyl), mono-or di-$C_{1-4}$ alkylamino and morpholino; and physiologically acceptable salts thereof.

Other sub-classes of compounds of formula (I) and their physiologically acceptable salts include those wherein:-

(i) $R^1$ is hydrogen;

(ii) $R^2$ is hydrogen;

(iii) one of $R^1$ and $R^2$ is hydrogen and the other is selected from carbamoyl, N-$C_{1-4}$ alkylcarbamoyl, carbamimidoyl, $N^1$-$C_{1-4}$ alkylcarbamimidoyl and imidazolin-2-yl; and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

provided that when n is 2 and $R^2$ is hydrogen, then

(a) $R^1$ is not carbamoyl when $R^3$ is hydrogen or methyl; and optionally

(b) $R^1$ is not N-methylcarbamoyl when $R^3$ is hydrogen; and

(iv) one of $R^1$ and $R^2$ is hydrogen and the other is a tetrazol-5-yl group optionally substituted by one or more $C_{1-4}$ alkyl groups; and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

with the optional proviso that:

when $R^1$ is unsubstituted tetrazol-5-yl, $R^2$ is hydrogen and $R^3$ is selected from hydrogen, chlorine, methyl, ethyl and t-butyl, then n is 1; and

when $R^1$ is 2-methyltetrazol-5-yl and $R^2$ and $R^3$ are both hydrogen, then n is 0 or 1.

Particularly preferred compounds of formula (I) and where appropriate, of formula (I') include the following compounds and where appropriate their physiologically acceptable salts, especially their hydrochlorides and dihydochlorides;

A. 3-(2-Imidazolin-2-yl)thioxanthen-9-one 10, 10-dioxide
B. 6-(2-Imidazolin-2-yl)-2-methoxythioxanthen-9-one 10, 10-dioxide
C. 2-Ethoxy-6-(2-imidazolin-2-yl)thioxanthen-9-one 10, 10-dioxide
D. 6-(2-Imidazolin-2-yl)-2-propoxythioxanthen-9-one 10, 10-dioxide
E. 6-(2-Imidazolin-2-yl)-2-fluorothioxanthen-9-one 10, 10-dioxide
F. 6-(2-Imidazolin-2-yl)-2-methylthioxanthen-9-one 10, 10-dioxide
G. 3-(2-Imidazolin-2-yl)-7-propylthioxanthen-9-one 10, 10-dioxide
H. 6-(2-Imidazolin-2-yl)-2-isopropylthioxanthen-9-one 10, 10-dioxide
I. 2-Ethylamino-6-(2-imidazolin-2-yl)thioxanthen-9-one 10, 10-dioxide
J. 2-Dimethylamino-6-(2-imidazolin-2-yl)thioxanthen-9-one 10, 10-dioxide
K. 6-(2-Imidazolin-2-yl)-2-(propylamino)thioxanthen-9-one 10, 10-dioxide
L. 2-(N-Ethylmethylamino)-6-(2-imidazolin-2-yl)thioxanthen-9-one 10, 10-dioxide
M. 2-Allylamino-6-(2-imidazolin-2-yl)thioxanthene-9-one 10, 10-dioxide
N. 2-(2-Aminoethylamino)-6-(2-imidazolin-2-yl)thioxanthen-9-one 10, 10-dioxide
O. 6-(2-Imidazolin-2-yl)-2-morpholinothioxanthen-9-one 10, 10-dioxide
P. 2-Chloro-6-(2-imidazolin-2-yl)thioxanthen-9-one 10, 10-dioxide
Q. N-Ethylcarbamoyl-7-propoxythioxanthen-9-one 10, 10-dioxide
R. 7-Isopropyl-N-methylcarbamoylthioxanthen-9-one 10, 10-dioxide
S. 3-N-methylcarbamoylthioxanthen-9-one 10, 10-dioxide
T. 7-isopropyl-3-(2-methyl)-2H-tetrazol-5-yl)thioxanthen-9-one 10,10-dioxide.
U. 3-(2-methyl-1H-tetrazol-5-yl)thioxanthen-9-one 10,10-dioxide.
V. 3-(1-methyl-1H-tetrazol-5-yl)thioxanthen-9-one 10, 10-dioxide.

Especially preferred is 3-N-methylcarbamoylthioxanthen-9-one 10, 10-dioxide. One metabolite of the latter compound is 3-carboxythioxanthen-9-one 10, 10-dixoide also known as thioxanthen-9-one 10,10-dixoide 3-carboxylic acid.

Some of the compounds of present invention have also been found to exhibit good MAO inhibitory activity whilst not producing significant hypertensive activity with orally ingested tyramine.

Thus the present invention further provides a compound of formula (I') as hereinbefore defined or a pharmacologically acceptable salt thereof, and pro-drugs and metabolites thereof, for use in a method of inhibiting monoamine oxidase-A in a mammal such as man, the method comprising administration to

said mammal of said compound, salt or pro-drug in an amount sufficient to inhibit the monoamine oxidase -A.

The invention also provides a compound of formula (I') as hereinbefore defined or a pharmacologically acceptable salt thereof, and pro-drugs and metabolites thereof, for use in a method of prophylaxis or treatment of a mental disorder in a mammal such as man, the method comprising administration to said mammal of a therapeutically effective amount of said compound salt or pro-drug.

In such a method the mental disorder may for example be:-

(a) depression, particularly that characterised by anxiety or obsessional neuroses, or an atypical depression, e.g. accompanied by a personality disorder;
(b) obssessive compulsive states; and
(c) anxiety states, eg which are accompanied in an acute phase by panic attacks.
(d) certain appetite disorders, eg. bulimia or anorexia.

The compounds of formula (I') and their physiologically acceptable salts and pro-drugs thereof may be administered by for example the oral, rectal or parenteral route. In general, the compound, may be administered at a dosage in the range of 2mg to 100 mg per kg of recipient bodyweight per day, although the precise dosage will naturally depend on a number of clinical factors, for example, the type (i.e. human or animal), age of the subject, the condition under treatment and its severity. For administration of the compounds by the oral route, a dosage regime of 1 to 50 mg per kg per day preferably 5 to 40, eg about 10mg per kg per day may be used, while for administration by the parenteral route, especially intravenously, a dosage regime of 0.2 to 10 mg per kg per day, advantageously 1 to 5 e.g. about 2 mg per kg per day is generally preferred. The compound may be administered intravenously by infusion, if desired, in which case, a dosage rate of, for example, 1-4 mg/min may be employed.

The compounds of formula (I'), their pharmacologically acceptable salts and pro-drugs are preferably administered in the form of pharmaceutical formulations.

The present invention thus further provides pharmaceutically acceptable formulations comprising as active ingredient, one or more compounds of formula (I') (as defined above) or physiologically acceptable salt(s) thereof, or pro-drugs

thereof, in association with at least one pharmaceutical carrier or excipient. The pharmaceutical formulations may be adapted for oral, parenteral (particularly intravenous) or rectal administration.

The formulations may conveniently be presented in unit dosage form and may be prepared by any of the methods well known in the art of pharmacy. Such methods include the step of bringing into association the active ingredient with the carrier which may comprise one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid or as an oil-in-water liquid emulsion or a water-in-oil liquid emulsion.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for rectal administration may be presented as a suppository with the usual carriers such as cocoa butter.

Formulations suitable for parenteral administration include aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze dried (lyophilized) condition requiring only the addition of the sterile liquid carrier, for example water for injections, immediately prior to use. Extemporaneous injection solutions and suspensions may be prepared from sterile powder, granules and tablets of the kind previously described.

Preferred unit dosage formulations are those containing a daily dose or unit daily sub-dose, as hereinabove recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The compounds of formula (I') and their physiologically acceptable salts may be synthesised by any method known in the art for synthesis of compounds of like and analogous structure, for example as described in the Batchelor and Hodson specifications, and in particular by the processes described hereinbelow.

Thus according to a further feature of the present invention we provide a process for the preparation of compounds of formula (I') (as hereinbefore defined) and their physiologically acceptable salts, the said process comprising:

a) reacting a compound of formula (II)

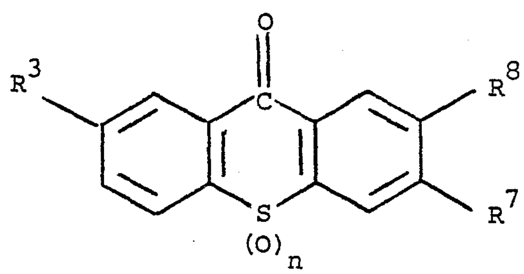

(II)

(wherein $R^3$ and n are as previously defined, and one of $R^7$ and $R^8$ is hydrogen and the other is a precursor for a group $R^1$ or $R^2$ respectively, as hereinbefore defined) with an agent or agents serving to effect conversion of the $R^7$ or $R^8$ group to the desired $R^1$ or $R^2$ group as appropriate; or

b) oxidising a compound of formula (III)

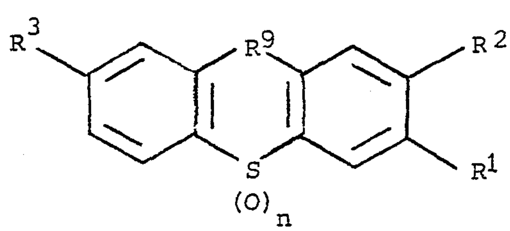

(III)

(wherein $R^1$-$R^3$ and n are as defined above and $R^9$ is a methylene group or a group of formula >-OH).

Where appropriate, in either of processes (a) and (b) above, the reactant compound of formula (II) or (III) may be provided in the form of a suitable salt thereof. The above processes, where appropriate, may also include one or more of the following optional steps:-

(i) Converting the resultant compound of formula (I') or salt thereof, or a precursor therefor, into a physiologically acceptable salt thereof.

(ii) Where the group $R^3$ in the resultant compound of formula (I') is a displaceable group, for example a halogen such as fluorine, converting the compound to another compound of formula (I') wherein $R^3$ is a group $OR^4$ or $-NR^5R^6$ as defined, or an amino-$C_{1-4}$ alkylamino group, by reaction with an appropriate alkoxide or amino compound, for example $NaOCH_3$ or $HN(CH_3)$ in a suitable solvent, e.g. methanol or dimethylformanide (DMF) preferably in the presence of a base. It will be appreciated that similar displacements may also be performed at various stages of the preparation of compounds of formula (I').

(iii) At an appropriate point in a synthetic route to a compound of formula (I'), protecting one or more functional groups (for example, converting the central carbonyl group to a ketal group) and in a later or final step, deprotecting in order to regenerate the desired group.

(iv) Where, in a compound of formula (I') or a physiologically acceptable salt thereof, n is 0 or 1, increasing the oxidation state of the sulphur atom so as to form repectively, a corresponding compound or salt wherein n is 1 or 2, or 2. Again, it will be appreciated that oxidation of the sulphur atom may be performed at any appropriate stage of the preparation.

(v) Where, in a compound of formula (I) or a physiologically acceptable salt thereof, $R^1$ or $R^2$ represents an unsubstituted carbamoyl or carbamimidoyl group, alkylating the said group to form a corresponding compound wherein $R^1$ or $R^2$ as appropriate, represents an N-$C_{1-4}$ alkylcarbamoyl, N,N-di-$C_{1-4}$ alkylcarbamoyl, $N^1$-$C_{1-4}$ alkylcarbamimidoyl, $N^2$-$C_{1-4}$alkylcarbamimidoyl, $N^1$-$C_{1-4}$ alkyl-$N^2$-$C_{1-4}$alkylcarbamimidoyl, $N^1$, $N^1$-di-$C_{1-4}$alkylcarbamimidoyl or $N^1$, $N^1$-di-$C_{1-4}$alkyl-$N^2$- $C_{1-4}$ alkylcarbamimidoyl group. Such alkylation may be performed by any of the methods well known to those skilled in the art,

for example by reaction with an alkyl-(eg.methyl-) halide, for example the iodide. A particular example of this conversion is the conversion of a carbamoyl group to an N-$C_{1-4}$ alkylcarbamoyl group, for example N-methylcarbamoyl.

(vi) Where, in a compound of formula (I') or a physiologically acceptable salt thereof, $R^1$ or $R^2$ represents an unsubstituted tetrazol-5-yl group, alkylating the said group to form a corresponding compound wherein $R^1$ or $R^2$ as appropriate, represents a $C_{1-4}$ alkyltetrazol-5-yl group. Such alkylation may be performed by methods as described in optional step (v) above.

For the preparation of compounds of formula (I') wherein one of $R^1$ and $R^2$ is hydrogen and the other is a carbamoyl, N-$C_{1-4}$ alklycarbamol or N, N-di-$C_{1-4}$alkylcarbamoyl group, process (a) above may comprise reaction of a compound of formula (II) wherein one of $R^7$ and $R^8$ is hydrogen and the other is an appropriate carbonyl derivative with one or more suitable reagents serving to effect amination of the said carbonyl derivative. Thus for example, where the $R^7$ or $R^8$ group represents an activated carbonyl group such as an ester or an acid chloride, the compound of formula (II) may be reacted with ammonia or a corresponding alkyl-or di-alkylamine, for example methylamine, preferably under aqueous conditions. If the carbonyl derivative is carboxy, the compound of formula (II) may be treated with a reagent serving to effect formation of a corresponding activated acid, in the presence of, or followed by reaction with an appropriate reagent such as described above in respect of reactions with esters and acid chlorides.

The reagent serving to effect formation of a corresponding activated acid may be sulphur oxychloride, a phosphorus halide such as phosphorus tri- or pentachloride, a phosphorus oxyhalide such as the oxychloride, trifluoroacetic anhydride, an alkyl- (eg. ethyl-) chloroformate or any other suitable agent which will be apparent to those skilled in the art. A list of such reagents appears in Methoden der organischen Chemie, Houben-Weyl, 4th Edn., Vol 15, 1, p.29. Conveniently, the reaction may be performed in a suitable solvent such as toluene, desirably in the presence of an appropriate catalyst such as dimethylformamide. Alternatively the reaction may be effected by reaction with an appropriate weak or volatile base such as methylamine, preferably by heating. Such a reaction conveniently may be effected using a stream of methylamine or by use of methylamine *in situ* in the presence of a dehydrating agent.

For the preparation of compounds of formula (I') wherein one of $R^1$ and $R^2$ is hydrogen and the other is a carbamimidoyl, $\underline{N}^1$-$C_{1-4}$ alkylcarbamimidoyl, $\underline{N}^2$-$C_{1-4}$alkylcarbamimidoyl, $\underline{N}^1$-$C_{1-4}$alkyl-$\underline{N}^2$-$C_{1-4}$alkylcarbamimidoyl, $\underline{N}^1$, $\underline{N}^1$-di-$C_{1-4}$ alkylcarbamimidoyl, $\underline{N}^1$,$\underline{N}^1$-di-$C_{1-4}$alkyl-$\underline{N}^2$- $C_{1-4}$alkylcarbamimidoyl, or optionally alkylated imidazolin-2-yl group, the precursor group may be a cyano group.

For the preparation of compounds of formula (I') wherein one of $R^1$ and $R^2$ is hydrogen and the other is an optionally alkylated tetrazol-5-yl group, in process (a), the compound of formula (II) may be reacted with hydrazoic acid the precursor and the reaction conditions may for example be as described in the Batchelor and Hodson specifications.

In process (b), the oxidation may be effected by reaction with hydrogen peroxide in acetic acid, with an organic peracid such as meta-chlorobenzoic acid in an inert solvent, eg. chloroform or dichloroethane, or with an inorganic peracid. Alternative oxidising agents for use in this stage of the process include ozone and alkali metal permanganates. Where the reaction is performed in an organic solvent, a crown ether is preferably included to ensure solution of the product. Other suitable inorganic oxidising agents include alkali metal chromates and dichromates in an unreactive solvent such as acetic acid. When it is also desired to perform the sulphur oxidation of option (iv) above and this is not brought about simultaneously with the oxidation of the 9-OH group in process (b), then it may be effected by admixture with a catalytic amount of strong base such as an alkali metal hydroxide or alkoxide, e.g. the t-butoxide, thereby promoting the air oxidation.

The following examples illustrate the present invention.

Example 1: Reference Preparation - 3-N-methylcarbamoylthioxanthen-9-one 10, 10-dioxide

To 345.2g (1.2mol) or 3-carboxy-10, 10-dioxothioxanthone was added 1.5Kg $SOCl_2$ and the mixture was refluxed overnight to convert the 3-carboxy group to the corresponding 3-carbonyl chloride, i.e., acid chloride. A further 500g of $SOCl_2$ was added and reflux was continued for another day. Excess $SOCl_2$ was removed under vacuum (water aspirator). The residue was cautiously added with cooling and stirring to 1500g of cold 70% aq. $NH_2CH_3$ and was stirred for 2 days. the precipitate was filtered, washed with aq. $NaHCO_3$ and dried to leave a solid residue, weight 328g. The procedure was repeated using another 200g of acid starting material.

The resultant solids were washed in $NaHCO_3$ solution, filtered and recrystallised from a mixture of EtOH and DMSO by addition of water. The crystals were filtered, washed with water and dried. The filtrate was diluted with water until cloudy, heated to solution and cooled to give addtional product. Further recrystallisation yielded 3-N-methylcarbamoylthioxanthene-9-one 10, 10-dioxide, m.p. 223-225°C; TLC on silica gel ($CHCl_3$: acetone/9:1) one spot $R_f$ 0.49.

Analysis: calc. for $C_{15}H_{11}NO_4S$; C, 59.79; H, 3.68; N, 4.65.
Found C, 59.74; H, 3.71; N, 4.62.

Example 2: 3-(2-Imidazolin-2-yl)thioxanthen-9-one 10, 10-dioxide (Compound A)

To 13.5 g (0.05 mol) of 3-cyanothioxanthen-9-one 10, 10-dioxide was added ethylenediamine, 6.0 g (0.1 mol), and sodium methoxide, 0.5 g (0.01 mol), in 100 ml methanol and the mixture was heated at reflux for 3 days. The reaction mixture was filtered hot and the solid was washed with two 50 ml portions of methanol. The crude product was recrystallized from ethanol to yield 3-(2-imidazolin-2-yl)thioxanthen-9-one 10, 10-dioxide, 11.0 g, as bright orange crystals, m.p. 241-242°C.

Analysis: Calc. for $C_{16}H_{12}N_2O_3S$; C, 61.52; H, 3.87; N, 8.97.
Found: C, 61.52; H, 3.78; N, 8.78.

Example 3-6

The following compounds were prepared by the procedure recited in Example 2 with appropriate cyano (nitrile) starting materials prepared according to the methods described in the Hodson and Batchelor specifications.

3. 6-(2-Imidazolin-2-yl)-2-methylthioxanthen-9-one 10, 10-dioxide (Compound F), m.p. 285-287°C.

4. 3-(2-Imidazolin-2-yl)-7-propylthioxanthen-9-one 10, 10-dioxide (Compound G), m.p. 200°C.

5. 6-(2-Imidazolin-2-yl)-2-isopropyl-9-thioxanthen-9-one 10, 10-dioxide (Compound H), m.p. 202°C.

6. 2-Chloro-6-(2-imidazolin-2-yl)thioxanthen-9-one 10, 10-dioxide (Compound P), m.p. 250-252°C.

Example 7: 6-(2-Imidazolin-2-yl)-2-methoxythioxanthen-9-one 10, 10-dioxide (Compound B)

a) 7-Fluoro-9-oxo-3-thioxanthenecarboxylic acid

The method described in the Hodson and Batchelor specifications was used in condensing 4-fluorothiophenol, 19.2 g (0.15 mol), with 2,4-dicyanonitrobenzene, 26.0 g (0.15 mol), in DMF in the presence of NaH to give 35 g of 2,4-dicyano-4' -fluorodiphenylsulfide. This sulfide, 34 g (0.13 mole), was heated at reflux in a KOH solution to hydrolyze the cyano groups to the corresponding carboxylic acid groups. The resulting diacid compound was cyclized in $H_2SO_4$ by heating at 80$^oC$ for an hour and the reaction mixture was poured into ice water to precipitate 7-fluoro-9-oxo-3-thioxanthenecarboxylic acid as yellow crystals. This material was collected, washed and dried to yield 22.2 g, m.p. 352$^oC$.

Analysis: calc. for $C_{14}H_7FO_3S$; C, 61.31; H, 2.57.
Found: C, 61.05; H, 2.59.

b) 7-Fluoro-9-oxo-3-thioxanthene carboxylic acid-10, 10-dioxide

To 256.78 g (0.936 mol) of 7-fluoro-9-oxo-3-thioxanthene carboxylic acid in 4.8 L of glacial acetic acid was added 528 ml of 30% $H_20_2$ at 75$^oC$. The mixture was refluxed 10 minutes, then an additional 125 ml of $H_20_2$ was added to the mixture, and refluxing was continued for 10 minutes. The reaction mixture was diluted with $H_20$ (2 L), chilled to 10$^oC$ and filtered. The solid was washed with 3 x 1 L of $H_20$ and dried, m.p., 325-330$^oC$; TLC on silica gel (butanol:acetic acid:water/5:4:1), $R_f$ = 0.69.

Analysis: calc. for $C_{14}H_7FO_5S$; C, 54.91; H, 2.30; S, 10.47.
Found: C, 54.96; H, 2.34; S, 10.47.

c) 7-Methoxy-9-oxothioxanthene-3-carboxylic acid 10, 10-dioxide

Fifteen g (0.049 mol) of 7-fluoro-9-oxo-3-thioxanthene carboxylic acid 10, 10-dioxide was dissolved in HMPA (hexamethylphosphoramide) (300 ml) and to this was added sodium methoxide, 6.75 g (0.13 mol), in HMPA, 225 ml.

The solution was stirred at 150°C for 2-5 hr and was then poured into ice water, 1 L. The mixture was acidfied with conc. HCl, diluted to 1.5 L and cooled overnight. The precipitate was filtered, dried and recrystallised from a mixture of EtOH, DMSO and $H_2O$ to yield 6.4 g of 7-methoxy-9-oxothioxanthene-3-carboxylic acid 10, 10-dioxide as an orange powder, m.p. 300-305°C; TLC on silica gel (ethanol), $R_f = 0.74$.

Analysis: calc. for $C_{15}H_{10}O_6S$; C, 56.60; H, 3.17.
Found: C, 56.63; H, 3.21.

d) 3-Cyano-7-methoxy-9-oxothioxanthene 10, 10-dioxide

A solution of 7-methoxy-9-oxothioxanthene-3-carboxylic acid 10, 10-dioxide, 6 g (0.019 mol), in thionyl chloride, 35 ml, was refluxed for 2.75 hr. The thionyl chloride was removed under reduced pressure, and the residue was stirred in concentrated $NH_4OH$ for 1.5 hr. The precipitate was filtered, washed with water and dried to afford the amide, 5.8g. The amide was added to a cooled (0°) solution of thionyl chloride 11 ml in DMF 36 ml, and the mixture was stirred at 0-10°C for 1 hr, then at room temperature for 1.5 hr. The mixture was poured into ice water (200 ml) and the resultant solid was filtered and dried (5.2 g); TLC on silica gel (hexane/ethylacetate: 7/2), $R_f = 0.59$.

e) 6-(2-Imidazolin-2-yl)-2-methoxythioxanthen-9-one-10, 10-dioxide

To 5.0 g (0.017 mol) of 6-cyano-2-methoxythioxanthen-9-one 10, 10-dioxide was added ethylenediamine, 10.8 g (0.18 mol), and sodium methoxide, (0.098 g, 0.002 mol), in methanol, 70 ml, and the mixture was refluxed for 70.5 hr. The mixture was then poured into ice water, 400 ml, and the precipitate was filtered and washed with water then was recrystallized from ethanol, DMSO and $H_2O$ to provide 1.1 g of 6-(2-imidazolin-2-yl)-2-methoxythioxanthen-9-one-10, 10-dioxide with m.p. 222-224°C; TLC on silica gel (ethanol), $R_f = 0.44$.
Analysis: calc. for $C_{17}H_{14}N_2O_4S$; C, 59.64; H, 4.12; N, 8.18.
Found: C, 59.83; H, 4.29; or 8.36.

Examples 8 and 9

The compounds of these two examples were prepared from 7-fluoro-9-oxo-3-thioxanthene carboxylic acid 10, 10-dioxide by the procedure of example 6.

8. 2-Ethoxy-6-(2-imidazolin-2-yl)thioxanthen-9-one 10, 10-dioxide (Compound C), m.p. 278-279°C (dec).

9. 6-(2-Imidazolin-2-yl)-2-propoxythioxanthen-9-one 10, 10-dioxide (Compound D), m.p. p. 157-160°C (dec).

Example 10: 6-(2-Imidazolin-2-yl)-2-fluorothioxanthene-9-one 10, 10-dioxide (Compound E)

a) 7-Fluoro-9-oxothioxanthene-3-carboxamide-10, 10-dioxide

A mixture of 7-fluoro-9-oxo-3-thioxanthene carboxylic acid 10, 10-dioxide (see example 6), 34.0 g (0.11 mol), and thionyl chloride, 130.9 g (1.10 mol), was heated at reflux for 2.0 hr. Dimethyl formamide, 1 ml, was added to the mixture, giving a solution which was heated at reflux overnight. The reaction was cooled, and a yellow crystalline solid, 27.85 g, was collected and washed with ether. A portion of the acid chloride, 5.0 g, was placed in concentrated $NH_4OH$, 50 mL, and heated on a steam bath for 0.5 hr. The solid was filtered and washed with $H_2O$ and acetone to give 3.82 g of the amide. A small amount of the amide was recrystallized from DMF:MeOH/75:25 to provide 0.3 g of a light yellow crystalline 7-fluoro-9-oxothioxanthene-3-carboxamide10, 10-dioxide, m.p. 307-310°C. TLC ($CHCl_3$/MeOH/9/1) $R_f = 0.45$.

Analysis: calc. for $C_{14}H_8FNO_4S$; C, 55.08; H, 2.64; N, 4.59; S, 10.50. Found: C, 55.13; H, 2.66; N, 4.61; S, 10.57.

b) 6-Cyano-2-fluorothioxanthene-9-one 10, 10-dioxide

Thionyl chloride (75 g) was cooled to 5°C and DMF (150 ml) was added dropwise and the temperature was maintained before 10°C. The amide prepared in a) supra, 19.1 g, (0.06 mol) was added and the mixture was allowed to warm to room temperature and was stirred for 2 hr. The mixture was poured onto 1 L of ice and was filtered. The solid was washed with $H_2O$, 2x300 ml and dried. The nitrile was dissolved in hot acetic acid (800 ml), treated with charcoal and filtered through a celite bed. The solution was cooled, and the crystals were collected by filtration and dried in vacuo to give 14.48 g of 6-cyano-2-fluorothioxanthene-9-one 10, 10-dioxide, m.p. = 260-262°C; TLC on silica gel (butanol:acetic acid: $H_2O$/5:4:1), $R_f = 0.82$.

Analysis: calc. for $C_{14}H_6FNO_3S$; C, 58.54; H, 2.11; N, 4.88; S, 11.16.
Found: C, 58.61; H, 2.14; N, 4.81; S, 11.20.

c) 6-(2-Imidazolin-2-yl)-2-fluorothioxanthen-9-one 10, 10-dioxide

A mixture of 6-cyano-2-fluorothioxanthen-9-one 10, 10-dioxide, 5.2 g (0.02 mol), ethylenediamine, 1.2 g (0.02 mol), and sodium methoxide, 0.08 g (0.0015 mol), in methanol, 76 ml, was refluxed for 4 days. Additional ethylenediamine, 1.2 g (0.02 mol), was added and reflux continued for 5 days. The mixture was poured into ice and the precipitate was filtered, dried and recrystallised from ethanol, DMSO and $H_2O$; affording 2.6 g of 6-(2-imidazolin-2-yl)-2-fluorothioxanthen-9-one 10, 10-dioxide, m.p. 248-249°C; TLC on silica gel (triethylamine; ethanol; hexane/1:1:2), $R_f = 0.8$.

Analysis: calc. for $C_{16}H_{11}FN_2O_3S$; C, 58.18; H, 3.36; N, 8.48.
Found: C, 57.88; H, 3.49; N, 8.60.

Example 11: 2-Dimethylamino-6-(2-imidazolin-2-yl)thioxanthen-9-one 10, 10-dioxide dihydrochloride (Compound J)

a) 6 Cyano-2-dimethylamino-thioxanthen-9-one 10, 10-dioxide

To 30 g (0.1 mol) of 7-fluoro-9-oxothioxanthene-3-carbonitrile 10, 10-dioxide (this compound is prepared by the method of example 6d, supra), was added anhydrous dimethylamine, 400 ml. The mixture was stirred at room temperature until the excess amine had evaporated. The residue was collected and washed with ether to give 39.3 g of the crude product. A small amount of 6 Cyano-2-dimethylaminothioxanthen-9-one 10, 10-dioxide was recrystallized from DMSO to give 2.4 g of red crystals with m.p. 291-294°C; TLC on silica gel (hexane: EtOAc/3:2), $R_f = 0.45$.
Analysis: calc. for $C_{16}H_{12}N_2O_3S$; 61.53; H, 3.87; N, 8.97.
Found: C, 61.49; H, 3.92; N, 8.93.

b) 2-Dimethylamino-6-(2-imidazolin-2-yl)thioxanthen-9-one 10, 10-dioxide dihydrochloride

A mixture of 6-cyano-2-dimethylaminothioxanthen-9-one 10, 10-dioxide, 2.0 g (0.006 mol), ethylenediamine, 3 g, 0.05 mol, and sodium methoxide, 0.1 g (0.002 mol) in methanol, 30 ml, was refluxed for 48 hr. The mixture

was poured into $H_2O$ and the resultant precipitate was dissolved in boiling in 1N HCl. This solution was cooled and the dihydrochloride salt was filtered and recrystallized from 95% ethanol to yield 2-dimethylamino-6-(2-imidazolin-2-yl)thioxanthen-9-one 10, 10-dioxide dihydrochloride 0.3 g with m.p. 330-335°C (dec); TLC on silica gel (ethylacetate:ethanol:acetic acid/80: 15:5), $R_f = 0.4$.

Analysis calc. for $C_{18}H_{17}N_3O_3S.2$ HCl; C,50.47; H, 4.47; N, 9.81.
Found: C, 50.25; H, 4.37; N, 9.70.

Examples 12-17

The compounds in the following examples were prepared by the method of Example 10, supra, substituting the appropriate amino compound in place of dimethylamine.

12. 2-Ethylamino-6-(2-imidazolin-2-yl)thioxanthen-9-one 10, 10-dioxide hydrochloride (Compound I), m.p. 284-286° (dec.).

13. 6-(2-Imidazolin-2-yl)-2-(propylamino)thioxanthene-9-one 10, 10-dioxide (Compound K), m.p. 105-107°C (dec).

14. 2-(N-Ethylmethylamino)-6-(2-imidazolin-2-yl)-thioxanthen-9-one 10, 10- dioxide hydrochloride (Compound L), m.p. above 300°C.

15. 2-Allylamino-6-(2-imidazolin-2-yl)-thioxanthen-9-one 10, 10-dioxide (Compound M), m.p. 176.5-177.5°C.

16. 6-(2-Imidazolin-2-yl)-2-morpholinothioxanthen-9-one 10, 10-dioxide isethionate (Compound O) m.p. 186-188°C.

17. 2-(2-Aminoethylamino)-6-(2-imidazolin-2-yl)thioxanthen-9-one 10, 10- dioxide dihydrochloride (Compound N) m.p. 278-282°C (dec).

Example 18: 3-Ethylcarbamoyl-7-propoxythioxanthen-9-one 10, 10-dioxide (Compound Q)

The title compound was prepared by the method of Example 7c followed by the reference method of Example 1, m.p. 183-185°C.

Example 19: 7-Isopropyl-3-methylcarbamoylthioxanthen-9-one 10, 10-dioxide (Compound R)

The title compound was prepared by the reference method of Example 1 from the corresponding acid prepared by the methods described in the Hodson and Batchelor specifications, m.p. 2-3-205$^{o}$C.

Example 20: Preparation of 3-N-methylcarbamoylthioxanthen-9-one 10,10-dioxide (Compound S)

To 345.2g (1.2mol) of 3-carboxy-10,10-dioxothioxanthone was added 1.5Kg $SOCl_2$ and the mixture refluxed overnight. A further 500g of $SOCl_2$ was added and reflux continued for a further day. Excess $SOCl_2$ was removed under vacuum (water aspirator). The residue was cautiously added with cooling and stirring to 1500g of cold 70% aq. $NH_2CH_3$ and stirred for 2 days. The precipitate was filtered, washed with aq. $NaHCO_3$ and dried to leave a solid residue, weight 328g. The procedure was repeated using a further 200g of acid starting material.

The resultant solids were washed in $NaHCO_3$ solution, filtered and recrystallised from EtOH, DMSO and water. The crystals were filtered, washed with water and dried. The filtrate was diluted with water until cloudy and heated to solution. Further recrystallisation yielded a solid m.p. 223-225$^{o}$C. Tlc ($CHCl_3$ : acetone/9:1) one spot $R_f$ 0.49. Analysis: calc. C 59.79, H 3.68, N 4.65; found C 59.74, H 3.71, N 4.62. Structure confirmed by NMR.

Example 21: 7-Isopropyl-3-(2-methyl-2H-tetrazol-5-yl)thioxanthen-9-one 10, 10-dioxide (Compound T)

To 7.0 g (0.02 mol) of 7-isopropyl-3-(tetrazol-5-yl)thioxanthen-9-one 10,10-dioxide (prepared by the methods of Hodson and Batchelor) was added sodium hydride, 1.0 of a 50% dispersion in mineral oil (0.08 mol), in dry DMF, 50 ml. The mixture was stirred at room temperature for 10 min., then $CH_3I$, 29.6 g, (0.21 mol), was added and the mixture was stirred for an additional 4.5 hr. The mixture was poured into $H_20$, and the yellow precipitate was collected by filtration then recrystallized from a mixture of ethanol, DMSO and $H_20$ to provide 5.0 g of yellow crystals of 7-Isopropyl-3-(2-methyl-2H-tetrazol-5-yl)thioxanthen-9-one 10, 10-dioxide with m.p. 219-221$^{o}$C; TLC on silica gel (hexane:ethylacetate/4:1), $R_f$ = 0.21.

Analysis: calc. for $C_{18}H_{16}N_4O_3S$; C, 58.68; H, 4.38; N, 15.21.
Found: C, 58.71; H, 4.41; N, 15.17.

Example 22: 3-(2-Methyl-1H-tetrazol-5-yl)thioxanthen-9-one 10,10 dioxide (Compound U)

The title compound was prepared according to the method described in Example 10 of US patent specification 3 905 989, mp. 204-205°C.

Example 23: 3-(1-Methyl-1H-tetrazol-5-yl)thioxanthen-9-one 10, 10 dioxide (Compound V)

The title compound was prepared in a manner analogous to that described in Example 2 above, m.p. 270-271°C.

In the following formulation examples, the 'active ingredient' may be any compound of formula (I') as hereinbefore defined or a physiologically acceptable salt thereof (or a pro-drug thereof), for example 3-N-methylcarbamoylthioxanthen-9-one 10,10-dioxide.

Example A - 100mg Compression Coated Tablet

| | | |
|---|---|---|
| Core | Active Ingredient | 100mg |
| | Starch B.P. | 25mg |
| | Magnesium Stearate B.P. | 2mg |
| Coating | Lactose B.P. | 320mg |
| | Starch B.P. | 50mg |
| | Gelatin B.P. | 6mg |
| | Magnesium Stearate B.P. | 4mg |

The active ingredient and starch were granulated with water and dried. Magnesium stearate was added to the dried granules. Lactose and starch were granulated with a 10% w/v aqueous solution of gelatin and dried. Magnesium stearate was added to the dried granules. The granulated core was compressed with the granulated coating in a conventional compression moulding machine.

Example B - 200mg Capsule

| | |
|---|---|
| Active Ingredient | 200mg |
| Lactose B.P. | 200mg |
| Talc B.P. | 40mg |

The active ingredient, lactose and talc were brought into intimate admixture with one another and 440mg of the resultant mixture was introduced into a size O hard gelatin capsule.

Example C - 100mg Capsule

| | |
|---|---|
| Active Ingredient | 100mg |
| Lactose | 100mg |
| Maize starch | 100mg |
| Magnesium Stearate | 10mg |

The ingredients were mixed together until homogeneous and 310mg of the resulting mixture filled into each hard gelatin capsule.

Example D - 500mg Tablet

| | |
|---|---|
| Active Ingredient | 500mg |
| Maize Starch | 100mg |
| Microcrystalline Cellulose | 75mg |
| Magnesium Stearate | 10mg |
| Granulated polyvinylpyrrolidone | 10% w/v in 50% w/v aqueous ethanol |

The Active Ingredient, Maize Starch and Microcrystalline Cellulose were mixed together, and granulated with the alcoholic Polyvinylpyrrolidone. The resulting granules were dried, and compressed to produce tablets, each tablet having a weight of approximately 690mg.

The active ingredient was dissolved in the bulk of the Water and then made up to volume and sterilised by filtration. The resulting solution was distributed into ampoules under aseptic conditions.

Example E - Suppository

| | |
|---|---|
| Active Ingredient | 200mg |
| Suppository Base | 1.8mg |

The active ingredient in fine powder form was dispersed into a little of the molten Suppository Base at $50^{o}C$. The dispersion was incorporated into the bulk of the base at the same temperature, allowed to cool at $42^{o}$-$45^{o}C$, poured into suitable 2g suppository moulds and allowed to set at $15^{o}$-$20^{o}C$. Suppository Bases were Massa Esterinum C and Witten H Suppository Compound.

Example F - Dispersible Tablet

| Per tablet | |
|---|---|
| Active Ingredient | 200.00mg |
| Maize Starch | 40.00mg |
| Primojel (Trade name: sodium starch glycollate (125µm powder)) | 50.00mg |
| Dicalcium Phosphate Dihydrate | 50.00mg |
| Sodium Carboxymethyl Cellulose | 2.00mg |
| Dioctyl Sodium Sulphosuccinate | 0.25mg |
| Sodium Saccharin | 5.00mg |
| Microcrystalline Cellulose | 50.00mg |
| Magnesium Stearate | 3.00mg |
| | 400.25mg |

The active ingredient, half of the Maize Starch, the Primojel and Dicalcium Phosphate were mixed together and then granulated with a solution of Sodium Carboxymethyl Cellulose, Dioctyl Sodium Sulphosuccinate and Sodium Saccharin in a suitable volume of 50% Ethyl Alcohol. The granules were dried, the remaining Maize Starch, the Microcrystalline Cellulose and the Magnesium Stearate were blended-in and the resulting mixture compressed into tablets each having a weight of 400.25mg.

Biological Activity

I. MONAMINE OXIDASE INHIBITION-In Vitro Inhibition

A. In Vitro Inhibition

MAO was assayed with [3H]serotonin (0.2 mM, 5 Ci/mol) and [14C] β-phenethylamine (10 μM, 3 Ci/mol) as substrates in a double-label assay (White and Glassman, J. Neurochem 29:987-97 1977).

For studies of the kinetic mechanism of inhibition, the above method was used, except that a single substrate (serotonin or tyramine) was varied over a 10-fold concentration range that included the $K_m$ concentration. MAO activity was determined in the absence and presence of the compound under test at each substrate concentration in duplicate assays.

MAO activity was determined in the absence and presence of the compound under test at each substrate concentration. Table I below lists $I_{50}$ values found for certain of the preferred compounds of formula (I). For comparison, the value is also quoted for the known compound (designated KC).

Table I

Inhibiton of MAO-A (In Vitro)

| Compound | $I_{50}$ (X10-$^{-6}$M) |
|---|---|
| H | 0.05 |
| J | 0.02 |
| L | 0.04 |
| O | 0.012 |
| E | 0.20 |
| S | 0.05 |
| KC | 0.06 |

Compound S produced a potent selective inhibition of MAO-A which was competitive vs. the substrates, serotonin or tyramine $K_i$ = 0.016 μM with serotonin as substrate.

B.In Vivo Inhibition

To determine MAO inhibition in brains and livers of rats pretreated with reversible inhibitors, it was necessary to use an assay procedure that minimized

dilution of the inhibitor. Thus, high concentrations of brain tissue homogenates were incubated for very short incubation times. For brain assays, initial tissue was 3-fold diluted into each assay. Because of the very high MAO activity in liver homogenates, further dilution of tissue was necessary in order to obtain reliable data. In this case, three different homogenate concentrations were assayed, and precent inhibition was extrapolated to zero tissue dilution. Substrate concentrations were not saturating, but were chosen relative to $K_m$ values for serotonin and phenethylamine in order to give an estimate of MAO-A and B, respectively.

Brains from pretreated male Sprague - Dawley rats (sacrificed 3 hours after oral dosing), were homogenized in a buffer consisting of 0.1 M potassium phosphate and 5% sucrose (pH 7.4) at a 1:1 tissue wt/buffer volume ratio, using a motorized Teflon/glass homogenizer. MAO-A and B were determined by incubating 100 µl of tissue homogenate with 50 µl of a double-label substrate mixture to give final concentrations of [3H] serotonin, 0.4 mM (5 Ci/mol); and [14C]β-phenethylamine, 20 µM (3 Ci/mol)]. For blank assays 100 µl portions of homogenate were pre-incubated at $37^{0}C$ with pargyline (4 mM) before substrate addition. Incubations were at $37^{0}C$ for 30 sec. Assay mixtures were then acidified and products extracted as in the above in vitro method. Liver tissue was homogenized in the above phosphate-sucrose buffer at a 1:5 tissue wt/buffer volume ratio. Portions (5, 10 and 50 µl) of each homogenate were assayed with 50 µl of the above double-label substrate mixture in a total assay volume of 150 µl 0.067 M potassium phosphate, pH 7.4. Blank assays included the same amounts of homogenate pre-incubated with 4 mM pargyline for 15 min at $37^{0}C$. After addition of substrates, mixtures were incubated at $37^{0}C$ for 20 sec, acidified, and products extracted as above. Percent inhibition for each liver homogenate was obtained by plotting % inhibition vs. reciprocal of tissue concentration and extrapolating back to zero dilution.

For the compound of the invention, the following results were obtained at 3 hr after oral dosing.

| Dose | Percentage Inhibition of MAO-A | |
|---|---|---|
| (mg/kg p.o.) | Brain | Liver |
| 12.5 | $37 \pm 16$ | $79 \pm 5$ |
| 25 | $49 \pm 10$ | $94 \pm 4$ |
| 50 | $78 \pm 2$ | $92 \pm 4$ |

RMW/MHD/29th November 1984

$ED_{80}$ values for the known compound and Compound S were both found to be 50 mg/kg p.o. for brain. There was no significant inhibition of MAO-B in either tissue. In other experiments with the compound of the invention, for an oral dose of 50 mg/Kg, inhibition was found to maximise within 3-7 hours and to be negligible at 24 hours after dosing, indicating reversibility of the in vivo inhibition.

## II. EFFECTS ON BLOOD PRESSURE RESPONSE TO ORAL TYRAMINE

Compound S was tested for effects on the pressor response induced by tyramine in a conscious, unrestrained rat model. The method involves direct measurement of arterial blood pressure from a cannula implanted in the carotid artery and exteriorized through a small incision in the back of the neck. Peak changes in the pressor response following tyramine (p.o.) administration in animals pretreated with the compound of the invention (p.o.) were compared to changes seen after tyramine in animals pretreated with the MAO inhibitor, phenelzine (p.o.), and control (water-treated) animals.

To compare effects at equipotent doses that are relevant to antidepressant activity, either Compound S or phenelzine was given in a single oral dose that produced approximately 80% inhibition of brain MAO-A by the time of tyramine administration. Under these conditions, MAO-A of liver was inhibited by 90% or more.

Rats treated with vehicle exhibited blood pressure elevations at relatively high doses of tyramine above 27 mg/kg. Phenelzine pretreatment increased the responsiveness to tyramine 3- to 10-fold, whilst Compound S did not cause a statistically significant increase in the pressor response to tyramine, except at the high oral dose 90mg/kg tyramine.

## III.ACTIVITY IN AN ANIMAL MODEL PREDICTIVE OF ANTIDEPRESSANT ACTIVITY- Prevention of Tetrabenazine-Induced Sedation in Mice

Charles River CD-1 male mice (18-22 gms) were pretreated orally with various doses of the known compound, Compound S or the reference standard, phenelzine, 60 min, 3 hr, or 5 hr before injection of tetrabenazine (35 mg/kg i.p.). This dose of tetrabenazine caused 90-100% of treated mice to remain motionless with marked blepharoptosis, even when placed into a novel environment. Thirty minutes after injection of tetrabenazine, each mouse was scored on an arbitrary scale from one to four according to Vernier et al., (in 1st

Hahnemann Symp. on Psychosomatic Medicine, J.H. Nodin and J.H. Moyer Eds., Lea and Febiger, Philadelphia, 1962, pp 683-90) for both depression of exploratory behaviour (1-4) and presence or absence of blepharoptosis (1-4). Ratings were converted to a percent of rating received by mice receiving amitriptyline prior to tetrabenazine administration. Rectal temperature was also determined 30 min after tetrabenazine.

90 minutes after oral treatment with the compound of the invention a dose-dependent prevention of tetrabenazine-induced sedation was obtained. The oral $ED_{50}$ for the prevention of tetrabenazine-induced sedation at this time period was 49 $\pm$ 10 mg/kg. The $ED_{50}$ values obtained at 3 and 5 hr after treatment with Compound S were essentially the same as those found at 90 min. For the known compound, an $ED_{50}$ was not reached by 100mg/kg p.o. However by extrapolation the $ED_{50}$ was estimated as 200mg/kg. For comparison, phenelzine had an oral $ED_{50}$ value of 16 $\pm$ 1 mg/kg.

## IV TOXICITY

Except for moderate hypothermia in mice at 1000mg/kg, p.o., no overt symptoms occurred in rats or mice at accute does up to 1000 mg/kg, p.o. Thus, the pharmacological index (ratio of no visible symptom dose to $ED_{50}$ for potential anti-depressant activity) is >20 in mice and >10 in rats accute oral dosing. No mortality occurred in rats or mice after acute i.p. doses of up to 1000 mg/kg. Moderate hypothermia and muscle tone decrease were observed at 500-1000 mg/kg, i.p.

Claims:-

1. A compound of formula (I)

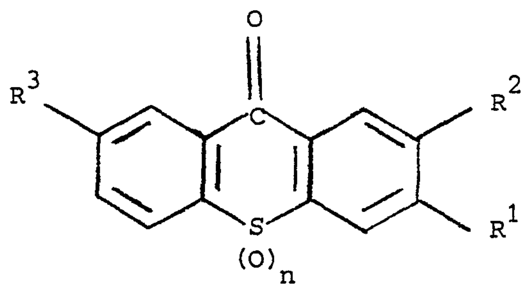

(I)

wherein n is 0, 1 or 2;

one of $R^1$ and $R^2$ is hydrogen and the other is selected from carbamoyl, N-$C_{1-4}$ alkylcarbamoyl, N,N-di-$C_{1-4}$alkylcarbamoyl, carbamimidoyl, $N^1$-$C_{1-4}$ alkylcarbamimidoyl, $N^2$-$C_{1-4}$alkylcarbamimidoyl, $N^1$-$C_{1-4}$alkyl-$N^2$-$C_{1-4}$alkyl-carbamimidoyl, $N^1$, $N^1$-di-$C_{1-4}$alkylcarbamimidoyl, $N^1$, $N^1$-di-$C_{1-4}$alkyl-$N^2$-$C_{1-4}$alkylcarbamimidoyl, tetrazol-5-yl (optionally substituted by one or more $C_{1-4}$ alkyl groups) and imidazolin-2-yl (optionally substituted by one or more $C_{1-4}$ alkyl groups); and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from hydrogen, $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

provided that when $R^1$ is carbamoyl, $R^2$ is hydrogen and $R^3$ is hydrogen or methyl, or when $R^1$ is 2-methyltetrazol-5-yl and $R^2$ and $R^3$ are both hydrogen, then n is 0 or 1; and

when $R^1$ is unsubstituted tetrazol-5-yl, $R^2$ is hydrogen and $R^3$ is selected from hydrogen, chlorine, methyl, ethyl and t-butyl, then n is 1;

or a physiologically acceptable salt thereof, and pro-drugs and metabolites thereof.

2. A compound as claimed in claim 1 wherein $R^1$ is hydrogen, $R^2$ represents an imidazolin-2-yl group or an N-$C_{1-4}$alkylcarbamoyl group (especially N-methylcarbamoyl) and $R^3$ represents a group selected from $C_{1-4}$ alkyl (especially isopropyl), mono-or di-$C_{1-4}$ alkylamino and morpholino; and physiologically acceptable salts thereof and pro-drugs and metabolites thereof.

3. A compound as claimed in claim 1 wherein one of $R^1$ and $R^2$ is hydrogen and the other is selected from carbamoyl, N-$C_{1-4}$ alkylcarbamoyl, carbamimidoyl, $N^1$-$C_{1-4}$ alkylcarbamimidoyl and imidazolin-2-yl; and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

provided that when n is 2 and $R^2$ is hydrogen, then

(a) $R^1$ is not carbamoyl when $R^3$ is hydrogen or methyl; and

(b) $R^1$ is not N-methylcarbamoyl when $R^3$ is hydrogen;

and physiologially acceptable salts thereof.

4. A compound as claimed in claim 1 wherein one of $R^1$ and $R^2$ is hydrogen and the other is a tetrazol-5-yl group optionally substituted by one or more $C_{1-4}$ alkyl groups; and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

provided that

when $R^1$ is unsubstituted tetrazol-5-yl, $R^2$ is hydrogen and $R^3$ is selected from hydrogen, chlorine, methyl, ethyl and t-butyl, then n is 1; and

when $R^1$ is 2-methyltetrazol-5-yl and $R^2$ and $R^3$ are both hydrogen, then n is 0 or 1; and physiologically acceptable salts thereof.

5. 3-N-methylcarbamoylthioxanthen-9-one 10, 10-dioxide.

6. A compound of formula (I')

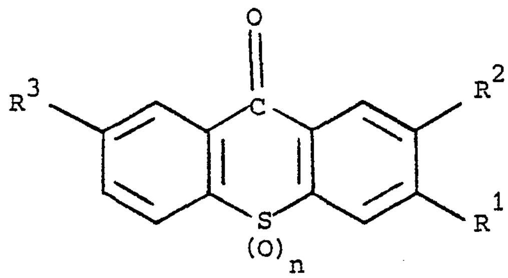

(I')

n is 0, 1 or 2;

one of $R^1$ and $R^2$ is hydrogen and the other is selected from carbamoyl, N-$C_{1-4}$ alkylcarbamoyl, N,N-di-$C_{1-4}$alkylcarbamoyl, carbamimidoyl, $N^1$-$C_{1-4}$ alkylcarbamimidoyl, $N^2$-$C_{1-4}$alkylcarbamimidoyl, $N^1$-$C_{1-4}$alkyl-$N^2$-$C_{1-4}$ alkylcarbamimidoyl, $N^1$, $N^1$-di-$C_{1-4}$alkylcarbamimidoyl, $N^1$, $N^1$-di-$C_{1-4}$alkyl-$N^2$- $C_{1-4}$alkylcarbamimidoyl, tetrazol-5-yl (optionally substituted by one or more $C_{1-4}$ alkyl groups) and imidazolin-2-yl (optionally substituted by one or more $C_{1-4}$ alkyl groups); and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from hydrogen, $C_{1-4}$ alkyl and hydroxy $C_{1-4}$ alkyl, provided that the total number of carbon atoms in $R^5$ and $R^6$ does not exceed 4), amino-$C_{1-4}$ alkylamino and morpholino;

provided that when $R^1$ is carbamoyl and $R^2$ and $R^3$ are both hydrogen, then n is 0 or 1;

or a physiologically acceptable salt thereof, or a pro-drug or metabolite of such a compound or salt, for use in the inhibition of monoamine oxidase-A in a mammal.

7. A compound of formula (I') as defined in claim 6 or a pro-drug or metabolite of such a compound or salt for use in a method of prophylaxis or treatment of a mental disorder in a mammal.

8. A compound, salt, pro-drug or metabolite as claimed in claim 7 wherein the mental disorder is selcted from depression, obsessive compulsive states, anxiety states and appetite disorders.

9. A compound, salt, pro-drug or metabolite as claimed in any of claims 6-8, wherein the mammal is man.

10. 3-carboxythioxanthen-9-one 10,10-dioxide for use as claimed in any of claims 6-8.

11. A pharmaceutical formulation comprising as active ingredient, one or more compounds of formula (I')

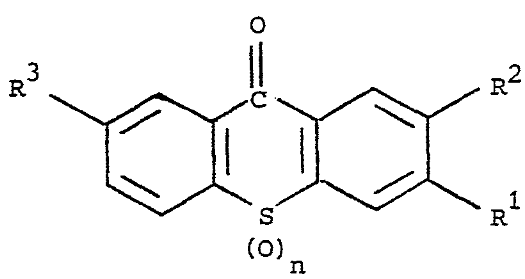

(I')

n is 0, 1 or 2;

one of $R^1$ and $R^2$ is hydrogen and the other is selected from carbamoyl, N-$C_{1-4}$ alkylcarbamoyl, N,N-di-$C_{1-4}$alkylcarbamoyl, carbamimidoyl, $N^1$-$C_{1-4}$ alkylcarbamimidoyl, $N^2$-$C_{1-4}$alkylcarbamimidoyl, $N^1$-$C_{1-4}$alkyl-$N^2$-$C_{1-4}$ alkylcarbamimidoyl, $N^1$, $N^1$-di-$C_{1-4}$alkylcarbamimidoyl, $N^1$, $N^1$-di-$C_{1-4}$alkyl-$N^2$- $C_{1-4}$alkylcarbamimidoyl, tetrazol-5-yl (optionally substituted by one or more $C_{1-4}$ alkyl groups) and imidazolin-2-yl (optionally substituted by one or more $C_{1-4}$ alkyl groups); and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from hydrogen, $C_{1-4}$ alkyl and hydroxy $C_{1-4}$ alkyl, provided that the total number of carbon atoms in $R^5$ and $R^6$ does not exceed 4), amino-$C_{1-4}$ alkylamino and morpholino;

provided that when $R^1$ is carbamoyl and $R^2$ and $R^3$ are both hydrogen, then n is 0 or 1.

or physiologically acceptable salts thereof, or pro-drugs thereof, in association with at least one pharmaceutical carrier or excipient.

12. A method of preparing a compound of formula (I)

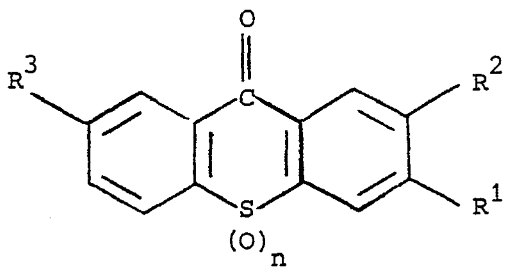

(I)

wherein n is 0, 1 or 2;

one of $R^1$ and $R^2$ is hydrogen and the other is selected from carbamoyl, N-$C_{1-4}$ alkylcarbamoyl, N,N-di-$C_{1-4}$alkylcarbamoyl, carbamimidoyl, $N^1$-$C_{1-4}$ alkylcarbamimidoyl, $N^2$-$C_{1-4}$alkylcarbamimidoyl, $N^1$-$C_{1-4}$ alkyl-$N^2$-$C_{1-4}$alkyl- carbamimidoyl, $N^1$, $N^1$-di-$C_{1-4}$alkylcarbamimidoyl, $N^1$, $N^1$-di-$C_{1-4}$alkyl-$N^2$- $C_{1-4}$alkylcarbamimidoyl, tetrazol-5-yl (optionally substituted by one or more $C_{1-4}$ alkyl groups) and imidazolin-2-yl (optionally substituted by one or more $C_{1-4}$ alkyl groups); and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently

selected from hydrogen, $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

provided that when $R^1$ is carbamoyl, $R^2$ is hydrogen and $R^3$ is hydrogen or methyl, or when $R^1$ is 2-methyltetrazol-5-yl and $R^2$ and $R^3$ are both hydrogen, then n is 0 or 1; and

when $R^1$ is unsubstituted tetrazol-5-yl, $R^2$ is hydrogen and $R^3$ is selected from hydrogen, chlorine, methyl, ethyl and t-butyl, then n is 1.

or a physiologically acceptable salt thereof, the method comprising

a) reacting a compound of formula (II)

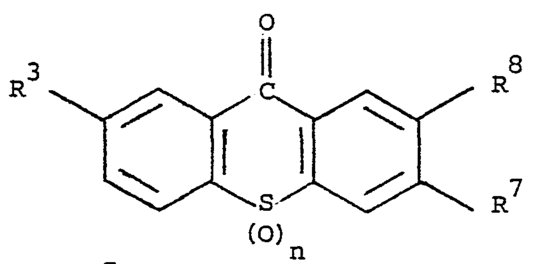

(II)

(wherein $R^3$ and n are as previously defined, and one of $R^7$ and $R^8$ is hydrogen and the other is a precursor for a group $R^1$ or $R^2$ respectively, as hereinbefore defined) with an agent or agents serving to effect conversion of the $R^7$ or $R^8$ group to the desired $R^1$ or $R^2$ group as appropriate; or

b) oxidising a compound of formula (III)

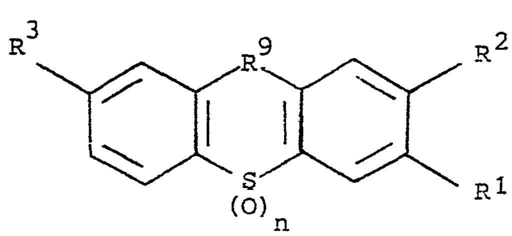

(III)

(wherein $R^1$-$R^3$ and n are as defined above and $R^9$ is a methylene group or a group of formula >-OH);

and where a compound of formula (I) is so prepared, optionally converting it to a physiologically acceptable salt thereof.

0150891

## Claims

1. A process for the preparation of compounds of formula (I)

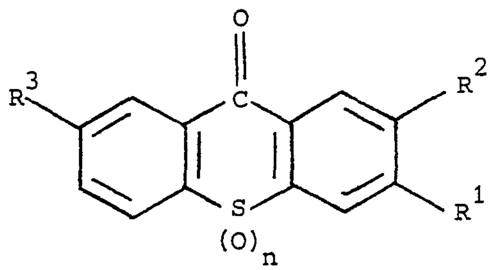

(I)

wherein n is 0, 1 or 2;

one of $R^1$ and $R^2$ is hydrogen and the other is selected from carbamoyl, N-$C_{1-4}$ alkylcarbamoyl, N,N-di-$C_{1-4}$alkylcarbamoyl, carbamimidoyl, $N^1$-$C_{1-4}$ alkylcarbamimidoyl, $N^2$-$C_{1-4}$alkylcarbamimidoyl, $N^1$-$C_{1-4}$alkyl-$N^2$-$C_{1-4}$alkylcarbamimidoyl, $N^1$, $N^1$-di-$C_{1-4}$alkylcarbamimidoyl, $N^1$, $N^1$-di-$C_{1-4}$alkyl-$N^2$-$C_{1-4}$alkylcarbamimidoyl, tetrazol-5-yl (optionally substituted by one or more $C_{1-4}$ alkyl groups) and imidazolin-2-yl (optionally substituted by one or more $C_{1-4}$ alkyl groups); and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from hydrogen, $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

provided that when $R^1$ is carbamoyl, $R^2$ is hydrogen and $R^3$ is hydrogen or methyl, or when $R^1$ is 2-methyltetrazol-5-yl and $R^2$ and $R^3$ are both hydrogen, then n is 0 or 1; and

when $R^1$ is unsubstituted tetrazol-5-yl, $R^2$ is hydrogen and $R^3$ is selected from hydrogen, chlorine, methyl, ethyl and t-butyl, then n is 1;
the method comprising

a) reacting a compound of formula (II)

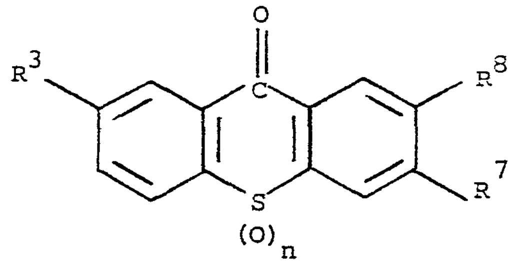

(II)

(wherein $R^3$ and n are as previously defined, and one of $R^7$ and $R^8$ is hydrogen and the other is a precursor for a group $R^1$ or $R^2$ respectively, as hereinbefore defined) with an agent or agents serving to effect conversion of the $R^7$ or $R^8$ group to the desired $R^1$ or $R^2$ group as appropriate; or

b) oxidising a compound of formula (III)

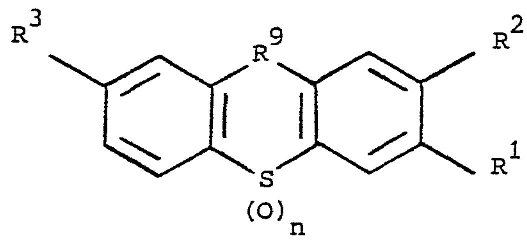

(III)

(wherein $R^1$-$R^3$ and n are as defined above and $R^9$ is a methylene group or a group of formula >-OH);

and optionally if desired, converting the compound of formula (I) so formed, to a physiologically acceptable salt thereof.

2. A process as claimed in claim 1, comprising one or more of the following optional additional steps:-

(i) where the group $R^3$ in the resultant compound of formula (I) is a displaceable group, for example a halogen such as fluorine, converting the compound to another compound of formula (I') wherein $R^3$ is a group $OR^4$ or $-NR^5R^6$ as defined, or an amino-$C_{1-4}$ alkylamino group, by reaction with an appropriate alkoxide or amino compound, for example $NaOCH_3$ or $HN(CH_3)$ in a suitable solvent, e.g. methanol or dimethylformanide (DMF) preferably in the presence of a base;

(ii) at an appropriate point in a synthetic route to a compound of formula (I), protecting one or more functional groups (for example,

converting the central carbonyl group to a ketal group) and in a later or final step, deprotecting in order to regenerate the desired group;

(iii) where, in a compound of formula (I) or a physiologically acceptable salt thereof, n is 0 or 1, increasing the oxidation state of the sulphur atom so as to form repectively, a corresponding compound or salt wherein n is 1 or 2, or 2;

(iv) where, in a compound of formula (I) or a physiologically acceptable salt thereof, $R^1$ or $R^2$ represents an unsubstituted carbamoyl or carbamimidoyl group, alkylating the said group to form a corresponding compound wherein $R^1$ or $R^2$ as appropriate, represents an N-$C_{1-4}$ alkylcarbamoyl, N,N-di-$C_{1-4}$ alkylcarbamoyl, $N^1$-$C_{1-4}$ alkylcarbamimidoyl, $N^2$-$C_{1-4}$alkylcarbamimidoyl, $N^1$-$C_{1-4}$alkyl-$N^2$-$C_{1-4}$alkylcarbamimidoyl, $N^1$, $N^1$-di-$C_{1-4}$alkylcarbamimidoyl or $N^1$, $N^1$-di-$C_{1-4}$alkyl-$N^2$-$C_{1-4}$ alkylcarbamimidoyl group; and

(v) where, in a compound of formula (I) or a physiologically acceptable salt thereof, $R^1$ or $R^2$ represents an unsubstituted tetrazol-5-yl group, alkylating the said group to form a corresponding compound wherein $R^1$ or $R^2$ as appropriate, represents a $C_{1-4}$ alkyltetrazol-5-yl group.

3. A process as claimed in claim 1 (a) comprising reaction of a compound of formula (II) wherein one of $R^7$ and $R^8$ is hydrogen and the other is an appropriate carbonyl derivative with one or more suitable reagents serving to effect amination of the said carbonyl derivative.

4. A process as claimed in claim 3 wherein the carbonyl derivative is carboxy and the compound of formula (II) may be treated with a reagent serving to effect formation of a corresponding activated acid, in the presence of, or followed by reaction with an appropriate reagent.

5. A process as claimed in claim 1 (b), wherein the oxidation is effected by reaction with hydrogen peroxide in acetic acid, with an organic peracid such as meta-chlorobenzoic acid in an inert solvent, or with an inorganic peracid.

6. A process as claimed in claim 1 or claim 2, wherein in the compound of formula (I) or physiologically acceptable salt so prepared, $R^1$ is hydrogen, $R^2$ represents an imidazolin-2-yl group or an N-

$C_{1-4}$alkylcarbamoyl group (especially N-methylcarbamoyl) and $R^3$ represents a group selected from $C_{1-4}$ alkyl (especially isopropyl), mono- or di-$C_{1-4}$ alkylamino and morpholino.

7. A process as claimed in claim 1 or claim 2, wherein in the compound of formula (I) or physiologically acceptable salt so prepared, one of $R^1$ and $R^2$ is hydrogen and the other is selected from carbamoyl, N-$C_{1-4}$ alkylcarbamoyl, carbamimidoyl, $N^1$-$C_{1-4}$ alkylcarbamimidoyl and imidazolin-2-yl; and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

provided that when n is 2 and $R^2$ is hydrogen, then

(a) $R^1$ is not carbamoyl when $R^3$ is hydrogen or methyl; and

(b) $R^1$ is not N-methylcarbamoyl when $R^3$ is hydrogen.

8. A process as claimed in claim 1 or claim 2, wherein in the compound of formula (I) or physiologically acceptable salt so prepared, one of $R^1$ and $R^2$ is hydrogen and the other is a tetrazol-5-yl group optionally substituted by one or more $C_{1-4}$ alkyl groups; and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from $C_{1-4}$ alkyl and hydroxy-$C_{1-4}$ alkyl, provided that the total number of carbon atoms in $R^5$ and $R^6$ does not exceed 4), amino-$C_{1-4}$ alkylamino and morpholino;

provided that:

when $R^1$ is unsubstituted tetrazol-5-yl, $R^2$ is hydrogen and $R^3$ is selected from hydrogen, chlorine, methyl, ethyl and t-butyl, then n is 1; and

when $R^1$ is 2-methyltetrazol-5-yl and $R^2$ and $R^3$ are both hydrogen, then n is 0 or 1.

9. A process as claimed in claim 1 or claim 2, wherein the compound of formula (I) so prepared is 3-N-methylcarbamoylthioxanthen-9-one 10, 10-dioxide.

10. A method of preparing a pharmaceutical formulation of a compound of formula (I')

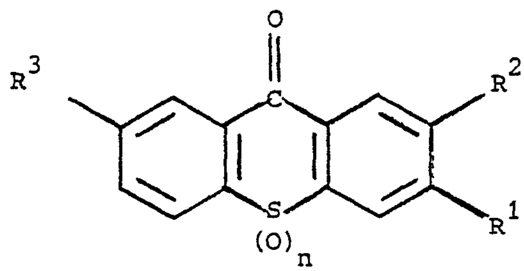

(I')

n is 0, 1 or 2;

one of $R^1$ and $R^2$ is hydrogen and the other is selected from carbamoyl, N-$C_{1-4}$ alkylcarbamoyl, N,N-di-$C_{1-4}$alkylcarbamoyl, carbamimidoyl, $N^1$-$C_{1-4}$ alkylcarbamimidoyl, $N^2$-$C_{1-4}$alkylcarbamimidoyl, $N^1$-$C_{1-4}$alkyl-$N^2$-$C_{1-4}$ alkylcarbamimidoyl, $N^1$, $N^1$-di-$C_{1-4}$alkylcarbamimidoyl, $N^1$, $N^1$-di-$C_{1-4}$alkyl-$N^2$- $C_{1-4}$alkylcarbamimidoyl, tetrazol-5-yl (optionally substituted by one or more $C_{1-4}$ alkyl groups) and imidazolin-2-yl (optionally substituted by one or more $C_{1-4}$ alkyl groups); and

$R^3$ is selected from hydrogen, saturated and unsaturated aliphatic hydrocarbon moieties containing from 1 to 4 carbon atoms, groups $OR^4$ (where $R^4$ is selected from hydrogen and saturated and unsaturated aliphatic hydrocarbon moieties containing from 1 to 4 carbon atoms), halo, groups of formula $-NR^5R^6$ (where $R^5$ and $R^6$ are independently selected from hydrogen, $C_{1-4}$ alkyl and hydroxy $C_{1-4}$ alkyl, provided that the total number of carbon atoms in $R^5$ and $R^6$ does not exceed 4), amino-$C_{1-4}$ alkylamino and morpholino;

provided that when $R^1$ is carbamoyl and $R^2$ and $R^3$ are both hydrogen, then n is 0 or 1;

characterised in that (i) one prepares a compound of formula (I') by

a) reacting a compound of formula (II)

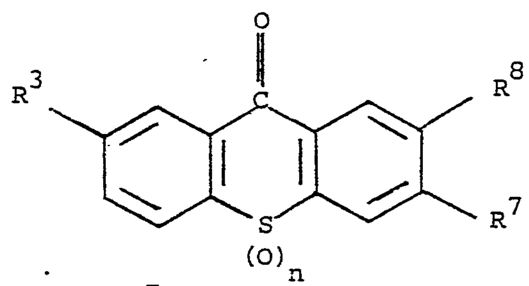

(II)

(wherein $R^3$ and n are as previously defined, and one of $R^7$ and $R^8$ is hydrogen and the other is a precursor for a group $R^1$ or $R^2$ respectively, as hereinbefore defined) with an agent or agents serving to effect conversion of the $R^7$ or $R^8$ group to the desired $R^1$ or $R^2$ group as appropriate; or

b) oxidising a compound of formula (III)

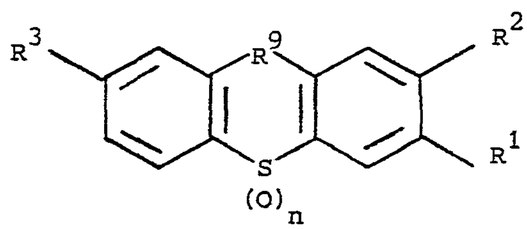

(III)

(wherein $R^1$-$R^3$ and n are as defined above and $R^9$ is a methylene group or a group of formula >-OH);

and optionally converts the compound of formula (I') so prepared to a physiologically acceptable salt thereof; and

(ii) one admixes the compound of formula (I') so prepared with a pharmaceutical carrier or excipient therefor.

0150891

European Patent Office

# PARTIAL EUROPEAN SEARCH REPORT

which under Rule 45 of the European Patent Convention shall be considered, for the purposes of subsequent proceedings, as the European search report

Application number

EP 85300011.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,A | US - A - 3 905 989 (HODSON)<br>* Formula I; column 1, lines 14-26 *<br>-- | 1 | C 07 D 335/16<br>C 07 D 409/04<br>A 61 K 31/38 |
| A | CH - A5 - 614 203 (THE WELLCOME FOUNDATION LTD)<br>* Claim 1 *<br>-- | 1 | |
| A | US - A - 4 101 558 (VACEK)<br>* Column 2, lines 46-66 *<br>-- | 1 | |
| A | CHEMICAL ABSTRACTS, vol. 99, no. 1, July 4, 1983, Columbus, Ohio, USA<br>J.F. BATCHELOR "Thioxanthones" page 504, column 2, abstract-no. 5518d<br>& Pat. Specif. (Aust.) AU 523 260, July 22, 1982<br>---- | 1 | |

TECHNICAL FIELDS SEARCHED (Int. Cl. 4)

C 07 D 335/00
C 07 D 409/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 3-5,10,12
Claims searched incompletely: 1,2,6-9,11
Claims not searched: -
Reason for the limitation of the search:

The terms "pro-drug" and "metabolite" do not define clearly the subject to be searched

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

| Place of search | Date of completion of the search | Examiner |
|---|---|---|
| VIENNA | 05-04-1985 | BRUS |

EPO Form 1505.1 06.78